# EUROPEAN PATENT SPECIFICATION

(11) **EP 1 913 974 B1**
(45) Date of publication and mention of the grant of the patent: **28.08.2013**
(21) Application number: 06753152.5
(22) Date of filing: 17.07.2006
(51) Int. Cl.: A61N 7/02, A61B 19/00

(54) **MRI-GUIDED FOCUSING ULTRASOUND THERAPEUTIC SYSTEM WITH HIGH INTENSITY**
MRT-GEFÜHRTES FOKUSSIERENDES ULTRASCHALLBEHANDLUNGSSYSTEM MIT HOHER INTENSITÄT
SYSTÈME THÉRAPEUTIQUE ULTRASONORE DE FOCALISATION GUIDÉ PAR IRM AYANT UNE INTENSITÉ ÉLEVÉE

(30) Priority: 29.07.2005 CN 200510088612
(43) Date of publication of application: 23.04.2008
(73) Proprietor: Chongqing Ronghai Medical Ultrasound Industry Ltd., Chongqing 401121 (CN)
(72) Inventor: WANG, Long, Chongqing 401121 (CN); GU, Yue, Chongqing 401121 (CN); MU, Mu, Chongqing 401121 (CN); FU, Bing, Chongqing 401121 (CN); WANG, Hai, Chongqing 401121 (CN)
(74) Representative: Cohausz & Florack
(86) International application number: PCT/CN2006/001715
(87) International publication number: WO 2007/012260

(56) References cited:
- EP-A2- 0 558 029
- EP-A2- 0 627 206
- CN-A- 1 185 982
- CN-A- 1 342 503
- US-A- 5 368 032
- US-A- 5 443 068
- US-A- 5 897 495

## Description

### FIELD OF THE INVENTION

The present invention pertains to the field of medical equipment, relates to an ultrasonic therapeutic system, and specifically to a MRI guided high-intensity focused ultrasonic therapeutic system.

### BACKGROUND OF THE INVENTION

The existing focused ultrasound therapies mainly use B-mode ultrasonic diagnostic apparatus to perform location and to monitor the therapy. Such B-mode ultrasonic diagnostic apparatus adopted in monitoring and treating has the following disadvantages: 1) Because B-mode ultrasound image is only a plane image with a certain angle and even though a 3-D ultrasound system is used, the visible area is still limited. 2) The ultrasound image is limited in the observation depth. The bone substances influence the image greatly and the tissue behind the bone almost cannot be displayed and the artifacts are existed. 3) Ultrasound images have poor capacity to identify the tissue boundary and particularly it is more difficult to identify small tumors and deep-bedded tumors.

Otherwise, a MRI (Magnetic Resonance Imaging) device is an important application in biological and medical fields. It may apply appropriate gradients to the magnetic field so that the magnetic resonance signals can be acquired selectively. The information is processed to gain the tissue characteristics of each point and further the tissue can be imaged. The acquired magnetic resonance images have a great ability to identify different tissues and are able to distinguish the normal tissue and tumor tissue easily. What we obtained are stereoscopic data within a certain volume and a part of human body or full body can be imaged, therefore MRI is very suitable to monitor the high-intensity focused ultrasound therapeutic procedure.

Japanese Patent JP3322649 discloses a therapy system combining a MRI device with an ultrasonic therapeutic equipment. Normally, the vertical moving distance under the treatment bed in the bore of MRI device is about 140 mm, therefore, the ultrasonic therapy equipment moves upward and downward for treatment in a quite limited space. Therefore, this therapy system employs MRI device to determine the location of a tumor firstly, and then the patient is moved out from the magnetic field of the MRI device and then treated by ultrasound. This kind of therapy system needs repeated moving of patient and needs location for many times. The locating system is complex and a long time is needed to make locations.

US Patent US5275165 provides a MRI surgery system, which facilitates surgery with a focused ultrasound transducer that selectively destroys tissues in a region within a patient. The focused ultrasound transducer focalizes energy at a focal point within the region of tissue to be destroyed. A non-magnetic moving and positioning device, whose moving mechanism is small enough to fit easily within the bore of the magnetic field of the MRI device, moves in a vertical direction. The moving and positioning device employs a plurality of hydraulic moving positioners and an inclined plane to drive the ultrasound transducer in a limited vertical space and positions the ultrasound focal point under the control of an operator. A MRI system employing a temperature sensitive pulse sequence creates an image of the tissue and the region being heated to allow the operator to adjust the position of the ultrasonic transducer so as to direct ultrasonic energy to the appropriate location. But the ultrasound transducer equipped in this moving and positioning device can not rotate and can only move from the bottom up for ultrasound treatment, therefore it can not meet the requirements of treating tumors in multiple orientations.

Another US Patent US5443068 similarly discloses a non-magnetic positioning device of an ultrasound energy applicator, which is operated within the bore of a MRI magnetic field. The purpose of this invention is to provide a simplified positioner, which is operated within the magnetic filed. And also the interference to the magnetic field of MRI system due to a moving means is avoided.

US Patent US6148225 with assignee of Siemens AG discloses an ultrasonic therapeutic apparatus, which adopts a high-frequency generator to generate discrete electrical signals with different frequency which developed within a frequency band in the order of time. Integral multiples of the discrete frequency values do not lie in a second frequency band that corresponds to the reception band of a simultaneously operated diagnostic MRI apparatus. In this way, the ultrasonic therapeutic apparatus does not interfere the magnetic field of the MRI apparatus when the ultrasound treatment is carried out during MRI monitoring.

All of above mentioned patents are directed to a therapy system combining a MRI device with an ultrasonic therapeutic equipment. These therapy systems mainly have the following disadvantages: 1) Because the vertical moving distance under the treatment bed in the bore of MRI is quite limited, it is very difficult to place an ultrasound transducer requiring a multiple-dimensional movement under the treatment bed and it is more difficult to place a complex moving and positioning mechanism. And the moving and positioning mechanism placed under the treatment bed in the bore of MRI occupies the limited space for moving an ultrasound transducer, accordingly the treatment procedure is affected; 2) The requirement of non-magnetic designs and treatment on the ultrasound transducer and its moving and positioning means for these systems are higher, therefore, the technical complexity and the cost of these systems are increased; 3) The ultrasound transducer driven by the moving and positioning mechanism can only move in a limited space and can not meet the multiple orientation requirement in treating diseases, like tumor. Because the ultrasound transducer can not rotate, it can only move from the bottom up for ultrasound treatment under the treatment bed. It can not work when ultrasound treatment need to be done both by side and from the top down; 4) It is difficult to perform assistant manual operations by an operator and also it is inconvenient for an operator to make clinical observations.

Document EP 0 627 026 A2 relates to an ultrasound medical treatment apparatus for treating a treatment target by irradiating intense focused ultrasound from outside of a patient, and an ultrasonic medical treatment apparatus utilizing imaging device.

### SUMMARY OT THE INVENTION

In view of the disadvantages of the related art as above mentioned, the technical problem to be solved by the present invention are to provide a MRI guided high-intensity focused ultarasonic therapeutic system, in which an ultrasound transducer moves in multiple orientations, freely moves without any limitation of space under the treatment bed, and brings less interference to a MRI apparatus.

The technical solution proposed by the present invention is: the MRI guided high-intensity focused ultrasonic therapeutic system includes a MRI apparatus and a high-intensity focused ultrasonic therapeutic apparatus. Said MRI apparatus includes the first treatment bed and said high-intensity focused ultrasonic therapeutic apparatus includes an ultrasound transducer and a moving and positioning means, which drives said transducer move in all directions. Wherein, said moving and positioning means is placed outside of the bore of the MRI apparatus during treatment and a supporting rod, which can extend into the bore of the MRI apparatus, is connected to said moving and positioning means at one end and is connected to said ultrasound transducer at the other end. Said moving and positioning means includes a control means, which drives said supporting rod to move in X, Y and Z-directions and rotate around X-direction. Driving by said control means, the supporting rod moves along X, Y and Z-directions and rotate around X-direction. Because said ultrasound transducer is connected to the supporting rod, the ultrasound transducer can be positioned precisely.

During the whole treatment procedure, said moving and positioning means isn't located in the bore of the MRI apparatus and is placed outside of the bore. Only through said supporting rod, said moving and positioning means is connected to said ultrasound transducer. In this way, the space under the treatment bed in the bore of the MRI apparatus is extended and the interference from the ultrasonic therapeutic apparatus to the magnetic field of MRI apparatus is decreased extremely. Taking full advantage of the structure and the function of the MRI apparatus, the present invention makes the area to be applied with energy by the ultrasound transducer intercross with the imaging and monitoring area of the MRI apparatus at the target area to be treated and achieves excellent therapeutic effects.

In present invention, said moving and positioning means may comprise an adjusting device for moving said moving and positioning means upward and downward. By adjusting that adjusting device, the supporting rod drives the ultrasound transducer to move in Z-direction in the bore of the MRI apparatus.

Said moving and positioning means may comprise a sliding device at its bottom for moving said moving and positioning means horizontally. The sliding device includes a pulley and a slide rail. The pulley is installed at the bottom of the moving and positioning means and is placed in the slide rail. When an operator applies force to the moving and positioning means in X-direction, the whole moving and positioning means driven by the pulley can move on the slide rail forward and backward and accordingly the ultrasound transducer driven by the supporting rod can move in X-direction in the bore of the MRI apparatus.

Said moving and positioning means may further comprise a rotating device for driving the supporting rod to rotate around the axis of MRI or the parallel lines of the axis. Thus, the ultrasound transducer can rotate around the axis of MRI or the parallel lines of the axis in the bore of MRI apparatus and variable and agile movement manners of the ultrasound transducer can be achieved for more appropriately meeting the needs of treatment.

In one preferable embodiment, the first treatment bed may be connected to the second treatment bed. The first treatment bed is connected to the second one by an adjustable fastening device. The adjustable fastening device can adjust the distance and angle between the two treatment beds freely and can drive the treatment beds to move in X-direction.

In another preferable embodiment, the MRI apparatus further comprises the second treatment bed and the moving and positioning means comprises a bed moving means for driving said second treatment bed to move rightward and leftward and forward and backward. The second treatment bed is connected to the bed moving means. Driven by the bed moving means, the second treatment bed may move rightward and leftward and forward and backward.

These additional devices mentioned as above facilitate an operator to assistantly manually operate the ultrasound transducer during treatment and are more helpful for an operator to make clinical observations during treatment.

Preferably, the supporting rod is made of non-magnetic material or non-metal material and accordingly the interference from the ultrasonic therapeutic apparatus to the magnetic field of the MRI apparatus is decreased further.

The ultrasound transducer can be placed within a container containing fluid. When the container connected to the supporting rod is placed under the first treatment bed or the second treatment bed, the first treatment bed or the second treatment bed has an aperture thereon and the ultrasound transducer can be placed in the appropriate position corresponding to the aperture of the first treatment bed or the second treatment bed. The container is an open one, which is open at the end faced to the aperture of the treatment bed and enables a patient to directly contact with the fluid in the container. When the container is placed above the first treatment bed, the container is a close one. The open end of the container is closed by an acoustically transparent membrane adapted to be in direct contact with the patient. Wherein, said fluid may be degassed water.

Because said moving and positioning means is placed outside of the bore of the MRI apparatus, the MRI guided high-intensity focused ultrasonic therapeutic system of the present invention minimizes the limitation on moving space of the ultrasound transducer and greatly reduces the non-magnetic requirements of the moving and positioning means of the ultrasound transducer and accordingly the most precise treatment on the nidus of the patient may be ensured. Meanwhile, the present invention integrates the MRI apparatus with the ultrasonic therapeutic apparatus better and increases the utilization rate of equipment. The present invention provides multiple movement manners for treatment and enables the medical personnel to treat the patient in various postures.

### BRIEF DESCRIPTION OF THE DRAWINGS

Fig. 1 is a schematic structural diagram of the embodiment 1 according to the present invention.
Fig. 2 is a schematic structural diagram of a high-intensity focused ultrasonic therapeutic apparatus in the embodiment 1 according to the present invention.
Fig. 3 is a schematic structural diagram of the embodiment 2 according to the present invention.
Fig. 4 is a schematic structural diagram of a second treatment bed in the embodiment 3 according to the present invention, which is placed outside of the MRI apparatus.
Fig. 5 is a schematic structural diagram of a second treatment bed in the embodiment 3 according to the present invention, which is placed in the MRI apparatus.
Fig. 6 is a schematic structural diagram of an adjustable fastening device in the embodiment 1 according to the present invention.

Wherein: 1 - MRI apparatus 2 - first treatment bed 3 - supporting means 4 - supporting rod 5 - container 6 - ultrasound transducer 7 - flexible matter 8 - rotating device 9-control means 10 - adjusting device 11, 31, 42, 43 - pulley 12, 20 - slide rail 13-second treatment bed 14 - adjustable fastening device 15 - acoustically transparent membrane 16-aperture 17, 23, 26, 41 - slide rail 18,21, 24, 27, 36, 39-motor 19-screw rod 22, 25, 40-screw rod 28, 37 - synchronization belt 29, 38 - synchronization belt-wheel 30 - transition piece 32 - pulley support 33 - bolt 34 - nut 35 - compressible spring

### DETAILED DESCRIPTION OF THE PREFERRED EMBODIMENTS

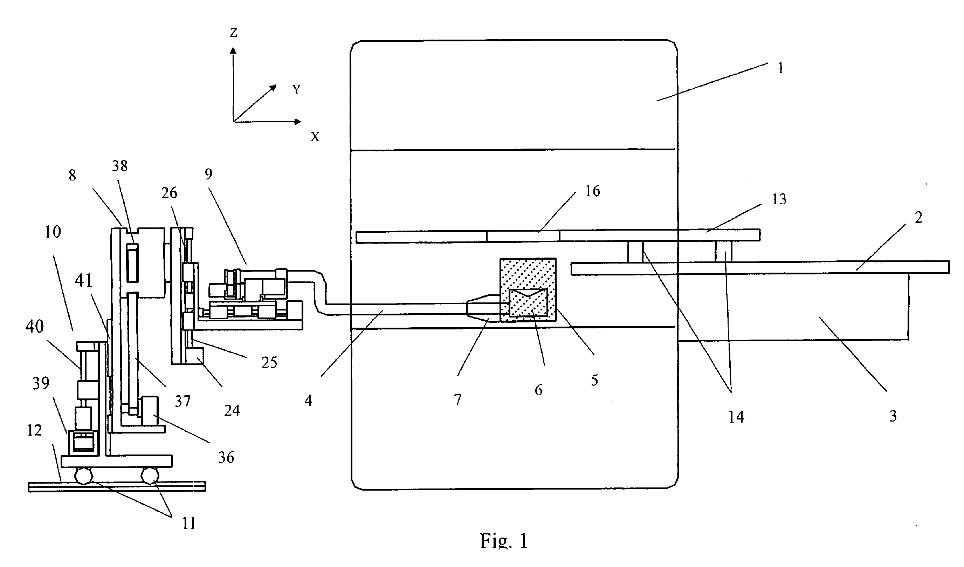

As shown in Figs. 1-5, a MRI guided high-intensity focused ultrasonic therapeutic system of the present invention comprises a MRI apparatus 1 and a high-intensity focused ultrasonic therapeutic apparatus.

A first treatment bed 2 in the bore of the MRI apparatus 1 is supported by a supporting means 3, which is fixed on the MRI apparatus 1. The high-intensity focused ultrasonic therapeutic apparatus comprises an ultrasound transducer 6 and a moving and positioning means for driving ultrasound transducer 6 to move in multiple-orientations and the moving and positioning means is placed outside of the bore of the MRI apparatus 1 and is connected to a supporting rod 4, which may extend into the bore of the MRI apparatus and is connected to the ultrasound transducer 6 at the other end.

The moving and positioning means comprises a control means 9 for driving supporting rod 4 to move in X, Y and Z-directions and rotate around X-direction. The moving and positioning means may comprise an adjusting device 10 for moving said moving and positioning means upward and downward. The moving and positioning means may comprise a sliding device at the bottom for moving the moving and positioning means horizontally. The sliding device includes a pulley 11 and a slide rail 12. The pulley 11 is installed at the bottom of moving and positioning means and the pulley 11 is placed on the slide rail 12. Said moving and positioning means may further comprise a rotating device 8 for driving the supporting rod to rotate around the axis of MRI or the parallel lines of the axis.

Preferably, supporting rod 4 is made of non-magnetic material or non-metal material.

The MRI guided high-intensity focused ultrasonic therapeutic system of the present invention further comprises the second treatment bed 13. The ultrasound transducer 6 is placed within a container 5, which is full of fluid and connected to the supporting rod 4. When the container 5 is placed under the treatment bed used by a patient (the first treatment bed or the second treatment bed), this treatment bed has an aperture 16 thereon. The position of the ultrasound transducer 6 corresponds to that of the aperture 16 of that treatment bed. The container 5 is an open one, which is open at the end faced to the aperture 16 and enables a patient to directly contact with the fluid in the container 5. When the container 5 is placed above the treatment bed used by a patient (the first treatment bed or the second treatment bed), the container is a close one. The open end of container 5 is closed by a flexible acoustically transparent membrane 15 adapted to be in direct contact with the patient. Wherein, the fluid may be degassed water.

The present invention is further described in detail taking in conjunction with the following embodiments and accompanying drawings.

### Embodiment 1:

As shown in Fig. 1, the system in this embodiment comprises a MRI apparatus 1 and a high-intensity focused ultrasonic therapeutic apparatus. Wherein, the high-intensity focused ultrasonic therapeutic apparatus comprises an ultrasound transducer 6, a moving and positioning means, which is located behind the MRI apparatus and is connected to a supporting rod 4. The supporting rod 4 is connected to a control means 9 of the moving and positioning means at its one end and is connected to ultrasound transducer 6 at the other end. The moving and positioning means comprises the control means 9, which drives the supporting rod 4 to move in X, Y and Z-directions and rotate around X-direction.

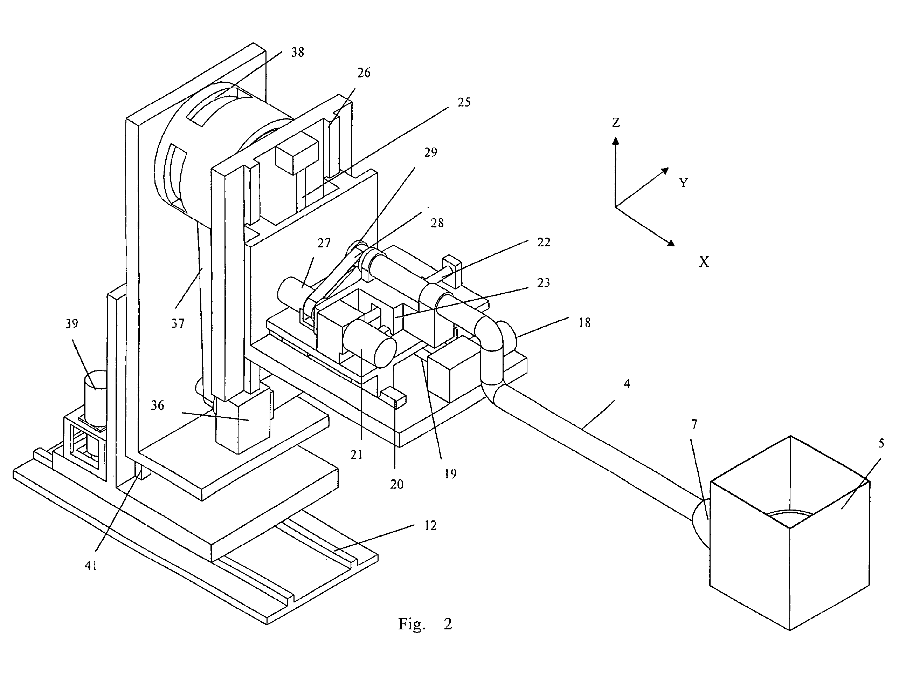

As shown in Fig. 1 and Fig. 2, each motion structure for movement in X, Y and Z directions of the control means 9 comprises a motor, a screw rod, a sliding block and a slide rail, which are integrated with the supporting rod. The screw rod is connected to the motor and the sliding block on the slide rail engages with the screw rod. Driven by a motor 18, a screw rod 19 drives the supporting rod 4 to move along a slide rail 20 in X-direction. Driven by a motor 21, a screw rod 22 drives the supporting rod 4 to move along a slide rail 23 in Y-direction. As shown in Fig. 1, driven by a motor 24, a screw rod 25 drives the supporting rod 4 to move along a slide rail 26 in Z-direction. The structure for rotating around X-direction of the control means 9 comprises a motor 27, a synchronization belt 28 and a synchronization belt-wheel 29. The synchronization belt-wheel 29 is integrated with the supporting rod 4. Through the synchronization belt 28, an output shaft of motor 27 is connected to the synchronization belt-wheel 29. Under the drive of the motor 27 and the force of the synchronization belt 28 and synchronization belt-wheel 29, the supporting rod 4 can rotate 0-360° around X-axis.

In this embodiment, the supporting rod 4 adopts a nickel-zinc-copper alloy in order to minimize the interference to the magnetic field of MRI apparatus 1.

As shown in Fig.1, there is a first treatment bed 2 and a second treatment bed 13 in the bore of the MRI apparatus 1 and the second one 13 is placed above the first one 2. The first treatment bed 2 is connected to the second one 13 by an adjustable fastening device 14, which can be used to adjust the position between the first treatment bed 2 and the second treatment bed 13. The first treatment bed 2 is fixed on a supporting means 3, which is fixed on the MRI apparatus 1. Wherein, there is an aperture on a section of the second treatment bed 13, which doesn't overlap with the first treatment bed 2.

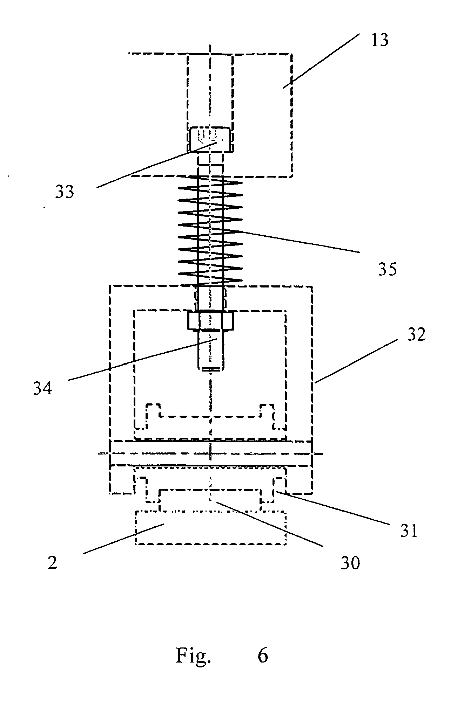

Fig. 6 is a schematic structural diagram of adjustable fastening device 14. The adjustable fastening device 14 comprises a transition piece 30, a pulley 31, a pulley support 32, a bolt 33, a nut 34 and a compressible spring 35. The transition piece 30 is embedded in the first treatment bed 2 and the pulley 31 is installed on the transition piece 30. The pulley support 32 is connected to a pulley shaft. The bolt 33 passes through the second treatment bed 13 and the pulley support 32 and is connected to the nut 34. The compressible spring 35 encases the bolt 33 and is sandwiched between the second treatment bed 13 and the pulley support 32. The transition piece 30 is embedded in the first treatment bed 2 so that the pulley 31 can move thereon. There are the bolt 33, the nut 34 and the compressible spring 35 installed between the pulley support 32 and the second treatment bed 13. When the second treatment bed 13 is pushed, under that pushing force, the second treatment bed 13 is driven by the pulley 31 and moves in X-direction. By adjusting the bolt 33 and the nut 34, the compressible spring 35 deforms and accordingly the distance between the two beds changes. Multiple sets of devices which include the bolt 33, the nut 34 and the compressible spring 35 can be installed between the two beds and by adjusting different bolts 33 and nuts 34, the compressible springs deform to different extents and accordingly the different angles can be formed between the two beds.

As shown in Fig.1 and Fig.2, the moving and positioning means further comprises a sliding device, which facilitates an operator to manually operate the ultrasound transducer during treatment. Said sliding device comprises a pulley 11 and a slide rail 12 for the pulley 11 sliding on.

As shown in Fig. 1, a rotating device 8 is connected to the control means 9 by a screw thread and the like. Under the drive of a motor 36 and the force of a synchronization belt 37 and a synchronization belt-wheel 38, the rotating device 8 drives the whole control means 9 to be rotatable 0-360° around the axis of MRI apparatus 1 or the parallel lines of the axis. Because the supporting rod 4 is connected to the control means 9, under the drive of the control means 9, the supporting rod 4 can also rotate 0-360° around the axis of MRI apparatus 1 or the parallel lines of the axis.

As shown in Fig. 2, the adjusting device 10 comprises a motor 39, a screw rod 40 and a slide rail 41. Under the drive of motor 39, the screw rod 40 drives the rotating device 8 to move upward and downward along the slide rail 41 in Z-direction. Because the control means 9 is connected to the rotating device 8 and the supporting rod 4 is connected to the control means 9, under the movement transmission, the supporting rod 4 can also move upward and downward in Z-direction.

The sliding device is located under the moving and positioning means and comprises the pulley 11 installed under the moving and positioning means and the slide rail 12 for the pulley 11 sliding on. When the force is applied to the moving and positioning means in X-direction, the whole moving and positioning means will move in X-direction.

After the ultrasound transducer 6 is positioned under the second treatment bed 13, the ultrasonic therapy can be applied to the nidus of the patient from the bottom up.

In this embodiment, the ultrasound transducer 6 is placed into a container 5, which contains fluid. The container 5 is a water bag, which is open at the end faced to the aperture 16 of the treatment bed 13. The container is full of degassed water. The degassed water is used as ultrasound propagation medium with a controlled temperature of about 25 °C. Because the ultrasound transducer 6 is placed in the water bag, the water bag is connected to the supporting rod 4 through a flexible matter 7. Wherein, ultrasound transducer 6 adopts spherical shell focusing piezoelectric transducer with a focal distance ranging from 80 mm to 200 mm, a diameter ranging from 80 mm to 300 mm and a working frequency ranging from 0.5 MHz to 2 MHz.

The work process of the therapy system in the present embodiment is: firstly, placing the patient on the second treatment bed 13 of the MRI apparatus 1, adjusting the supporting means 3 and/or the adjustable fastening device 14 to position the nidus of the patient within the magnet resonance volume and using the MRI apparatus 1 to image the nidus of the patient. Then, driving the supporting rod 4 by the moving and positioning means to move the ultrasound transducer 6 in the space under the second treatment bed 13, moving the ultrasound transducer 6 until it aims at the aperture 16 of the second treatment bed 13, and overlapping the focus of the ultrasound transducer 6 with the nidus of the target area to be treated within the magnetic resonance volume. Finally, transmitting the therapeutic ultrasonic waves and applying therapy to the patient.

### Embodiment 2:

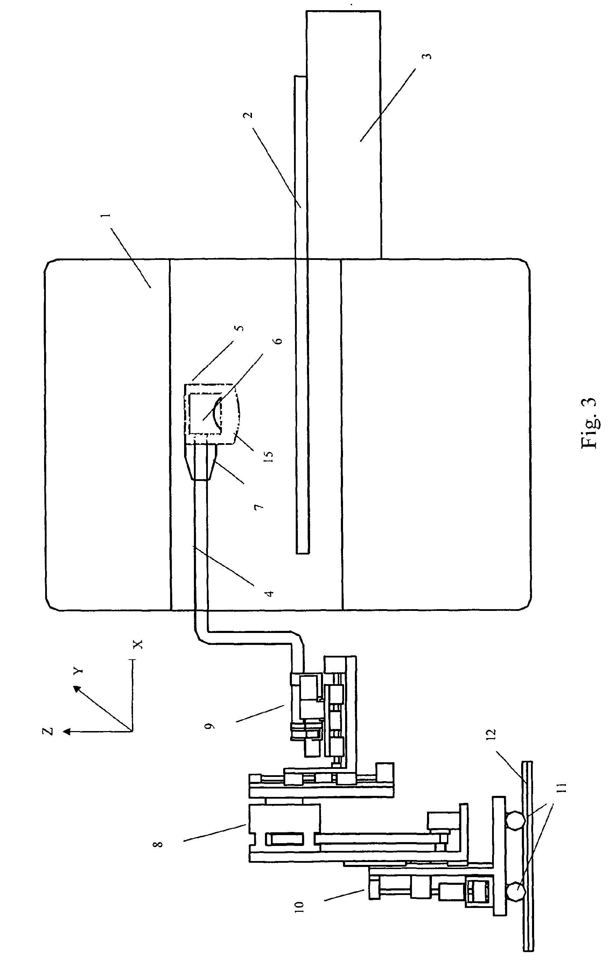

As shown in Fig. 3, in this embodiment, the moving and positioning means is located behind the MRI apparatus 1. It is different from the embodiment 1 that in this embodiment, there is only the first treatment bed 2 in the bore of the MRI apparatus 1 and the ultrasound transducer 6 is located above the first treatment bed 2 to apply the ultrasonic waves to the nidus of the patient from the top down. Because the ultrasound transducer 6 is located above the first treatment bed 2 to apply the ultrasonic waves to the nidus of the patient from the top down, the first treatment bed 2 in the MRI apparatus 1 may not have an aperture and it may be used for performing normal treatment.

The container 5 adopts a water bag, which is full of degassed water. In order to avoid the overflow of the degassed water in that bag, a flexible acoustically transparent membrane 15 is used to seal the open end of the water bag and that membrane can be in direct contact with the patient.

Other components in this embodiment are the same as those in the embodiment 1.

### Embodiment 3:

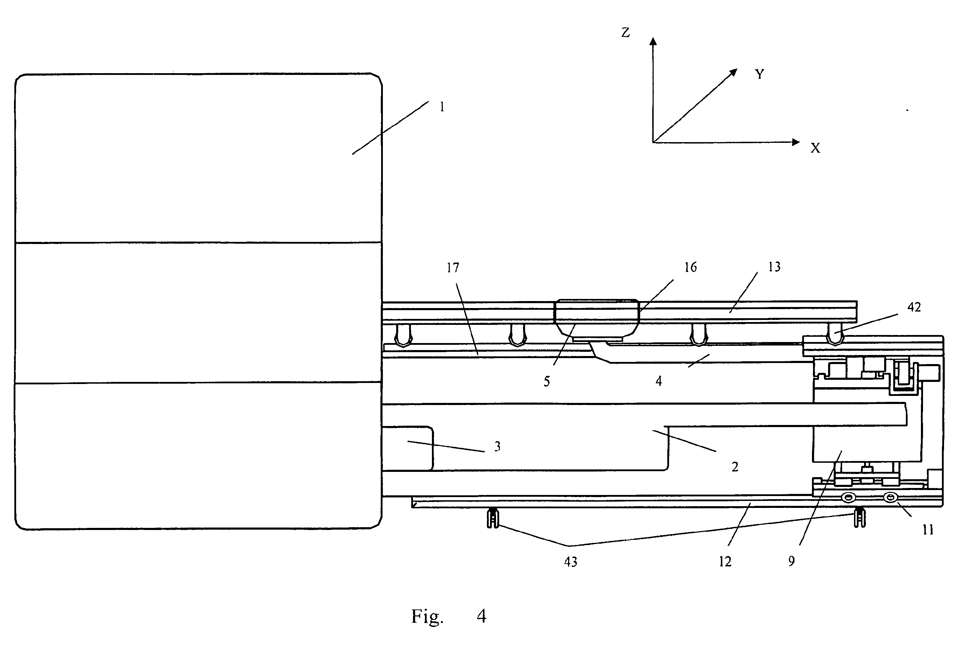

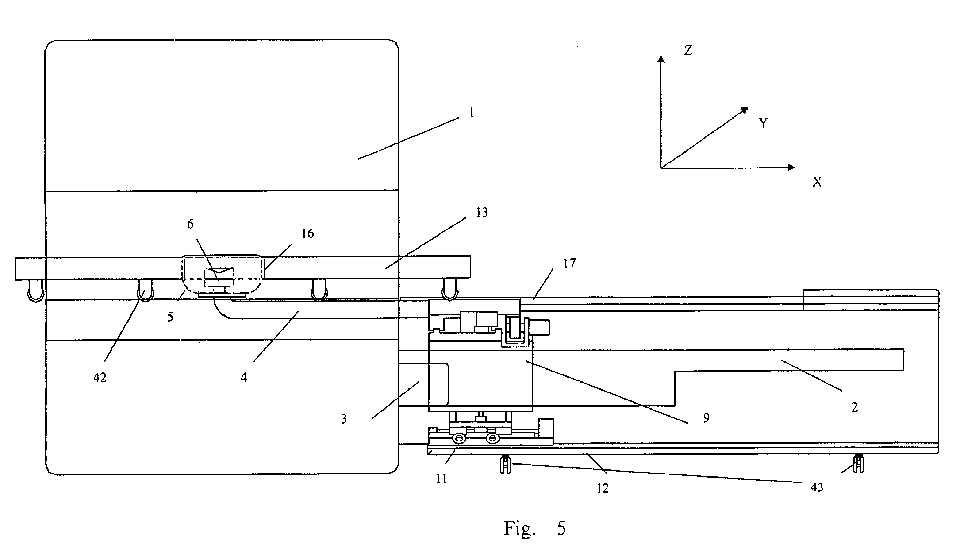

As shown in Fig. 4 and Fig. 5, in this embodiment, the moving and positioning means is in front of the MRI apparatus 1 during treatment. The MRI apparatus comprises a first treatment bed 2 and a second treatment bed 13. The moving and positioning means comprises a bed moving means for moving the second treatment bed 13 and a control means 9 for driving an ultrasound transducer to move in X, Y and Z-directions and rotate around X-direction. The second treatment bed 13 is connected to the moving and positioning means through the bed moving means.

Wherein, the structure of the control means 9 is the same as that in the embodiment 1.

The bed moving means comprises a structure of pulley and slide rail for moving the second treatment bed 13 rightward and leftward and a structure of pulley and slide rail for moving the second treatment bed 13 forward and backward.

As shown in Fig. 4, a slide rail 17 is fixed on the control means 9 and a fluid container 5 is placed in an aperture of the second treatment bed 13. When the whole high-intensity focused ultrasonic therapeutic apparatus is pushed by hand, under the driving of a pulley 43, the whole apparatus moves rightward and leftward along a slide rail 12 horizontally and is located in front of the MRI apparatus 1. As shown in Fig. 5, when the second treatment bed 13 is pushed by hand, the pulley 42 moves along the slide rail 17 forward and backward. Because the second treatment bed 13 is connected to the moving and positioning means, the whole moving and positioning means also moves along the slide rail 12 forward and backward by this force. And finally, the second treatment bed 13 enters into the bore of the MRI apparatus 1 and is positioned in the optimal position for treatment on the patient.

After the ultrasound transducer 6 is positioned under the second treatment bed 13, the ultrasonic therapy can be applied to the nidus of the patient from the bottom up.

Other components and functions in this embodiment are the same as those in the embodiment 1.

## Claims

1. An MRI guided high-intensity focused ultrasonic therapeutic system, comprising:
an MRI apparatus (1) which includes a first treatment bed (2) and has a bore; and
a high-intensity focused ultrasonic therapeutic apparatus which includes an ultrasound transducer (6) and a moving and positioning means, which drives said transducer (6) to move in respective directions,
wherein said moving and positioning means is placed outside of the bore of the MRI apparatus (1) during treatment,
wherein a supporting rod (4), which is extendable into the bore of the MRI apparatus (1), is connected to said moving and positioning means at one end and is connected to said ultrasound transducer (6) at the other end,
wherein said moving and positioning means includes a control means (9), for driving said supporting rod (4) to move in X, Y and Z-directions and for rotating around X-direction,
**characterized by**
a second treatment bed (13) connected to said first treatment bed (2) , and
the second treatment bed (2) being connected to the first one (13) by an adjustable fastening device (14).

2. An MRI guided high-intensity focused ultrasonic therapeutic system, comprising:
an MRI apparatus (1) which includes a first treatment bed (2) and has a bore; and
a high-intensity focused ultrasonic therapeutic apparatus which includes an ultrasound transducer (6) and a moving and positioning means, which drives said transducer (6) to move in respective directions,
wherein said moving and positioning means is placed outside of the bore of the MRI apparatus (1) during treatment,
wherein a supporting rod (4), which is extendable into the bore of the MRI apparatus (1), is connected to said moving and positioning means at one end and is connected to said ultrasound transducer (6) at the other end,
wherein said moving and positioning means includes a control means (9), for driving said supporting rod (4) to move in X, Y and Z-directions and for rotating around X-direction,
**characterized in that**
said MRI apparatus (1) further comprises a second treatment bed (13),
said moving and positioning means comprises a bed moving means for moving said second treatment bed (13) rightward and leftward and forward and backward and
the second treatment bed (13) is connected to said bed moving means.

3. The system as cited in claim 1 or 2, **characterized in that**
each motion structure for movement in X, Y and Z directions of the control means (9) comprises a motor (18, 21, 24, 27, 36, 39), a screw rod (19), a sliding block and a slide rail, which are integrated with the supporting rod (4), and
the screw rod (19) is connected to the motor (18, 21, 24, 27, 36, 39) and engages with the sliding block on the slide rail.

4. The system as cited in claim 1 or 2, **characterized in that**
the structure for rotating around X-direction of the control means (9) comprises a motor (18, 21, 24, 27, 36, 39), a synchronization belt (28, 37) and a synchronization belt-wheel (29, 38),
the synchronization belt-wheel (29, 38) is integrated with the supporting rod (4), and
through the synchronization belt (28, 37), an output shaft of the motor (18, 21, 24, 27, 36, 39) is connected to the synchronization belt-wheel (29, 38).

5. The system as cited in claim 1 or 2, **characterized in that**
said moving and positioning means further comprises an adjusting device (10) for moving said moving and positioning means upward and downward.

6. The system as cited in claim 1 or 2, **characterized in that**
said moving and positioning means comprise a sliding device at its bottom for moving said moving and positioning means horizontally.

7. The system as cited in claim 6, **characterized in that**
said sliding device includes a pulley (11, 31, 42, 43) and a slide rail (12, 20), and
the pulley (11, 31, 42, 43) is installed at the bottom of the moving and positioning means and is placed on the slide rail (12, 20).

8. The system as cited in claim 1 or 2, **characterized in that**
said moving and positioning means further comprise a rotating device (8) for driving the supporting rod to rotate around the axis of MRI or the parallel lines of the axis.

9. The system as cited in claim 1, **characterized in that**
said adjustable fastening device (14) comprises a transition piece (30), a pulley, a pulley support (32), a bolt (33), a nut (34) and a compressible spring (35), the transition piece (30) is embedded in the first treatment bed (2) and the pulley is installed on the transition piece (30),
the pulley support (32) is connected to the pulley shaft, the bolt (33) passes through the second treatment bed (13) and the pulley support (32) and is connected to the nut (34), and
the compressible spring (35) encases the bolt (33) and is sandwiched between the second treatment bed (13) and the pulley support (32).

10. The system as cited in claim 1 or 2, **characterized in that**
said supporting rod (4) is made of non-magnetic material or non-metal material.

11. The system as cited in claim 10, **characterized in that**
said ultrasound transducer (6) is placed within a container (5), which is full of fluid and connected to supporting rod (4),
the container (5) is placed under the first treatment bed (2) or the second treatment bed (13),
the first treatment bed (2) or the second treatment bed (13) has an aperture (16),
the container (5) is an open one, which is open at the end faced to the aperture (16) of treatment bed (2, 16), or
when the container (5) is placed above the first treatment bed (2), the container (5) is a close one, that is, the open end of the container (5) is closed by an acoustically transparent membrane (15).

12. The system as cited in claim 11, **characterized in that** said fluid is degassed water.

13. The system as cited in claim 10, **characterized in that** said ultrasound transducer (6) adopts piezoelectric transducer with a focal distance ranging from 80 mm to 200 mm, a diameter ranging from 80 mm to 300 mm and a working frequency ranging from 0.5 MHz to 2 MHz.

## Patentansprüche

1. MRI-gelenktes therapeutisches System mit hochintensivem fokussierten Ultraschall, umfassend:
eine MRI-Vorrichtung (1), die ein erstes Behandlungsbett (2) umfasst und eine Bohrung aufweist; und
eine therapeutische Vorrichtung mit hochintensivem fokussierten Ultraschall, die einen Ultraschallwandler (6) und ein Bewegungs- und Positionierungsmittel, das den Wandler (6) zur Bewegung in entsprechende Richtungen antreibt, umfasst,
wobei das Bewegungs- und Positionierungsmittel während der Behandlung außerhalb der Bohrung der MRI-Vorrichtung (1) angeordnet ist,
wobei eine Trägerstange (4), die in die Bohrung der MRI-Vorrichtung (1) ausfahrbar ist, mit dem Bewegungs- und Positionierungsmittel an einem Ende verbunden ist und mit dem Ultraschallwandler (6) am anderen Ende verbunden ist, wobei das Bewegungs- und Positionierungsmittel ein Steuermittel (9) zum Antreiben der Trägerstange (4) umfasst, so dass sich diese in X-, Y- und Z-Richtungen bewegt und um die X-Richtung dreht,
**gekennzeichnet durch**
ein zweites Behandlungsbett (13), das mit dem ersten Behandlungsbett (2) verbunden ist, und
wobei das zweite Behandlungsbett (13) **durch** eine einstellbare Befestigungsvorrichtung (14) mit dem ersten Behandlungsbett (2) verbunden ist.

2. MRI-gelenktes therapeutisches System mit hochintensivem fokussierten Ultraschall, umfassend:
eine MRI-Vorrichtung (1), die ein erstes Behandlungsbett (2) umfasst und eine Bohrung aufweist; und
eine therapeutische Vorrichtung mit hochintensivem fokussierten Ultraschall, die einen Ultraschallwandler (6) und ein Bewegungs- und Positionierungsmittel, das den Wandler (6) zur Bewegung in entsprechende Richtungen antreibt, umfasst,
wobei das Bewegungs- und Positionierungsmittel während der Behandlung außerhalb der Bohrung des MRI-Apparates (1) angeordnet ist,
wobei eine Trägerstange (4), die in die Bohrung der MRI-Vorrichtung (1) ausfahrbar ist, mit dem Bewegungs- und Positionierungsmittel an einem Ende verbunden ist und mit dem Ultraschallwandler (6) am anderen Ende verbunden ist, wobei das Bewegungs- und Positionierungsmittel ein Steuermittel (9) zum Antreiben der Trägerstange (4) umfasst, so dass sich diese in X-, Y- und Z-Richtungen bewegt und um die X-Richtung dreht,
**dadurch gekennzeichnet, dass**
die MRI-Vorrichtung (1) des Weiteren ein zweites Behandlungsbett (13) umfasst,
das Bewegungs- und Positionierungsmittel ein Bettbewegungsmittel zum Bewegen des zweiten Behandlungsbetts (13) nach rechts und links und nach vorne und rückwärts umfasst und
das zweite Behandlungsbett (13) mit dem Bettbewegungsmittel verbunden ist.

3. System nach Anspruch 1 oder 2, **dadurch gekennzeichnet, dass**
jede Bewegungsstruktur zur Bewegung in X-, Y- und Z-Richtungen des Steuermittels (9) einen Motor (18, 21, 24, 27, 36, 39), eine Schraubenspindel (19), einen Gleitblock und eine Gleitschiene umfasst, die mit der Trägerstange (4) integriert sind, und
die Schraubenspindel (19) mit dem Motor (18, 21, 24, 27, 36, 39) verbunden ist und mit dem Gleitblock auf der Gleitschiene in Eingriff steht.

4. System nach Anspruch 1 oder 2, **dadurch gekennzeichnet, dass**
die Struktur zum Drehen um die X-Richtung des Steuermittels (9) einen Motor (18, 21, 24, 27, 36, 39), einen Synchronisierungsriemen (28, 37) und ein Synchronisierungsriemenrad (29, 38) umfasst,
wobei das Synchronisierungsriemenrad (29, 38) mit der Trägerstange (4) integriert ist und
durch den Synchronisierungsriemen (28, 37) eine Abtriebswelle des Motors (18, 21, 24, 27, 36, 39) mit dem Synchronisierungsriemenrad (29, 38) verbunden ist.

5. System nach Anspruch 1 oder 2, **dadurch gekennzeichnet, dass**
das Bewegungs- und Positionierungsmittel des Weiteren eine Einstellvorrichtung (10) zum Bewegen des Bewegungs- und Positionierungsmittels nach oben und nach unten umfasst.

6. System nach Anspruch 1 oder 2, **dadurch gekennzeichnet, dass**
das Bewegungs- und Positionierungsmittel eine Gleitvorrichtung an seinem Boden zum horizontalen Bewegen des Bewegungs- und Positionierungsmittels umfasst.

7. System nach Anspruch 6, **dadurch gekennzeichnet, dass** die Gleitvorrichtung eine Rolle (11, 31, 42, 43) und eine Gleitschiene (12, 20) umfasst und
die Rolle (11, 31, 42, 43) am Boden des Bewegungs- und Positionierungsmittels installiert und auf der Gleitschiene (12, 20) angeordnet ist.

8. System nach Anspruch 1 oder 2, **dadurch gekennzeichnet, dass**
das Bewegungs- und Positionierungsmittel des Weiteren eine Drehvorrichtung (8) umfasst, die die Trägerstange in eine Drehung um die Achse des MRI oder um die parallelen Achsenlinien versetzt.

9. System nach Anspruch 1, **dadurch gekennzeichnet, dass** die einstellbare Befestigungsvorrichtung (14) ein Übergangsstück (30), eine Rolle, eine Rollenhalterung (32), einen Bolzen (33), eine Mutter (34) und eine zusammenpressbare Feder (35) umfasst, wobei das Übergangsstück (30) im ersten Behandlungsbett (2) eingebettet ist und die Rolle auf dem Übergangsstück (30) installiert ist,
wobei die Rollenhalterung (32) mit der Rollenwelle verbunden ist, der Bolzen (33) durch das zweite Behandlungsbett (13) geht und die Bolzenhalterung (32) mit der Mutter (34) verbunden ist und
die zusammenpressbare Feder (35) den Bolzen (33) umschließt und zwischen dem zweiten Behandlungsbett (13) und der Rollenhalterung (32) liegt.

10. System nach Anspruch 1 oder 2, **dadurch gekennzeichnet, dass**
die Trägerstange (4) aus einem nicht magnetischen Material oder einem nicht metallischen Material gebildet ist.

11. System nach Anspruch 10, **dadurch gekennzeichnet, dass** der Ultraschallwandler (6) in einem Behälter (5) angeordnet ist, der vollständig mit einem Fluid gefüllt und mit der Trägerstange (4) verbunden ist,
der Behälter (5) unter dem ersten Behandlungsbett (2) oder dem zweiten Behandlungsbett (13) angeordnet ist,
das erste Behandlungsbett (2) oder das zweite Behandlungsbett (13) eine Öffnung (16) aufweist,
der Behälter (5) ein offener ist, der an dem Ende offen ist, das der Öffnung (16) des Behandlungsbettes (2, 16) zugewandt ist, oder
wenn der Behälter (5) über dem ersten Behandlungsbett (2) angeordnet ist, der Behälter (5) ein geschlossener ist, das heißt, das offene Ende des Behälters (5) durch eine akustisch durchlässige Membrane (15) geschlossen ist.

12. System nach Anspruch 11, **dadurch gekennzeichnet, dass** das Fluid entgastes Wasser ist.

13. System nach Anspruch 10, **dadurch gekennzeichnet, dass** der Ultraschallwandler (6) einen piezoelektrischen Wandler mit einer Brennweite im Bereich von 80 mm bis 200 mm, einem Durchmesser im Bereich von 80 mm bis 300 mm und einer Arbeitsfrequenz im Bereich von 0,5 MHz bis 2 MHz anwendet.

## Revendications

1. Système thérapeutique à ultrasons concentrés à haute intensité guidé par IRM, comprenant :
un appareil d'IRM (1) qui inclut un premier lit de traitement (2) et qui a une ouverture ; et
un appareil thérapeutique à ultrasons concentrés à haute intensité qui inclut un transducteur ultrasonore (6) et un moyen de déplacement et de positionnement, qui entraîne le dit transducteur (6) pour qu'il se déplace dans les directions respectives,
où ledit moyen de déplacement et de positionnement est placé hors de l'ouverture de l'appareil d'IRM (1) pendant le traitement,
où une tige de support (4), qui est extensible dans l'ouverture de l'appareil d'IRM (1), est raccordée audit moyen de déplacement et de positionnement à une extrémité et est raccordée audit transducteur ultrasonore (6) à l'autre extrémité,
où ledit moyen de déplacement et de positionnement inclut un moyen de contrôle (9), pour entraîner ladite tige de support (4) à se déplacer dans les directions X, Y et Z et pour faire pivoter la direction X,
**caractérisé par**
un second lit de traitement (13) qui est raccordé audit premier lit de traitement (2), et
le second lit de traitement (13) étant raccordé au premier (13) par un dispositif de fixation réglable (14).

2. Système thérapeutique à ultrasons concentrés à haute intensité guidé par IRM, comprenant :
un appareil IRM (1) qui inclut un premier lit de traitement (2) et qui a une ouverture ; et
un appareil thérapeutique à ultrasons concentrés à haute intensité qui inclut un transducteur ultrasonore (6) et un moyen de déplacement et de positionnement, qui entraîne le dit transducteur (6) pour qu'il se déplace dans les directions respectives,
où ledit moyen de déplacement et de positionnement est placé hors de l'ouverture de l'appareil IRM (1) pendant le traitement,
où une tige de support (4), qui est extensible dans l'ouverture de l'appareil d'IRM (1), est raccordée audit moyen de déplacement et de positionnement à une extrémité et est raccordée audit transducteur ultrasonore (6) à l'autre extrémité, où ledit moyen de déplacement et de positionnement inclut un moyen de contrôle (9), pour entraîner ladite tige de support (4) à se déplacer dans les directions X, Y et Z et pour faire pivoter la direction X,
**caractérisé en ce que**
ledit appareil d'IRM (1) comprend en outre un second lit de traitement (13),
où ledit moyen de déplacement et de positionnement comprend un moyen de déplacement de lit pour déplacer le second lit de traitement (13) vers la droite et vers la gauche et vers l'avant et vers l'arrière et
le second lit de traitement (13) est raccordé audit moyen de déplacement du lit.

3. Système tel que cité à la revendication 1 ou 2, **caractérisé en ce que**
chaque structure de déplacement pour le déplacement dans les directions X, Y et Z du moyen de contrôle (9) comprend un moteur (18, 21, 24, 27, 36, 39), une tige à vis (19), un coulisseau et un rail glissière, qui sont intégrés avec la tige de support (4), et
la tige à vis (19) est raccordée au moteur (18, 21, 24, 27, 36, 39) et s'enclenche avec le coulisseau sur le rail glissière.

4. Système tel que cité à la revendication 1 ou 2, **caractérisé en ce que**
la structure servant à pivoter autour de la direction X du moyen de contrôle (9) comprend un moteur (18, 21, 24, 27, 36, 39), une courroie de synchronisation (28, 37) et une poulie de synchronisation (29, 38),
la poulie de synchronisation (29, 38) est intégrée avec la tige de support (4), et
par la courroie de synchronisation (28, 37), un arbre de sortie du moteur (18, 21, 24, 27, 36, 39) est raccordé à la poulie de synchronisation (29, 38).

5. Système tel que cité à la revendication 1 ou 2, **caractérisé en ce que**
ledit moyen de déplacement et de positionnement comprend en outre un dispositif de réglage (10) pour déplacer ledit moyen de déplacement et de positionnement vers le haut et vers le bas.

6. Système tel que cité à la revendication 1 ou 2, **caractérisé en ce que**
ledit moyen de déplacement et de positionnement comprend un dispositif de glissière dans sa partie inférieure pour déplacer ledit moyen de déplacement et de positionnement horizontalement.

7. Système tel que cité à la revendication 6, **caractérisé en ce que**
ledit dispositif de glissière inclut une poulie (11, 31, 42, 43) et un rail glissière (12, 20), et
la poulie (11, 31, 42, 43) est installée au fond du moyen de déplacement et de positionnement et est placée sur le rail glissière (12, 20),

8. Système tel que cité à la revendication 1 ou 2, **caractérisé en ce que**
ledit moyen de déplacement et de positionnement comprend en outre un dispositif pivotant (8) pour entraîner la tige de support autour de l'axe de l'IRM ou des lignes parallèles de l'axe.

9. Système tel que cité à la revendication 1, **caractérisé en ce que**
ledit dispositif de fixation réglable (14) comprend une pièce de transition (30), une poulie, un support de poulie (32), un boulon (33), un écrou (34) et un ressort compressible (35), la pièce de transition (30) est encastrée dans le premier lit de traitement (2) et la poulie est installée sur la pièce de transition (30),
le support de poulie (32) est raccordé à l'arbre de la poulie, le boulon (33) passe par le second lit de traitement (13) et le support de poulie (32) et est raccordé à l'écrou (34), et
le ressort compressible (35) entoure le boulon (33) et est serré entre le second lit de traitement (13) et le support de poulie (32).

10. Système tel que cité à la revendication 1 ou 2, **caractérisé en ce que**
ladite tige de support (4) est faite de matériau non-magnétique ou de matériau non-magnétique.

11. Système tel que cité à la revendication 10, **caractérisé en ce que**
ledit transducteur ultrasonore (6) est placé dans un récipient (5), qui est rempli de liquide et raccordé à la tige de support (4),
le récipient (5) est placé sous le premier lit de traitement (2) ou sous le second lit de traitement (13),
le premier lit de traitement (2) ou le second lit de traitement (13) a une ouverture (16),
le récipient (5) est un récipient ouvert, qui est ouvert sur l'extrémité faisant face à l'ouverture (16) du lit de traitement (2, 16), ou
quand le récipient (5) est placé au-dessus du premier lit de traitement (2) le récipient (5) est un récipient fermé, c'est-à-dire que l'extrémité ouverte du récipient (5) est fermée par une membrane acoustiquement transparente (15).

12. Système tel que cité à la revendication 11, **caractérisé en ce que** ledit liquide est de l'eau dégazée.

13. Système tel que cité à la revendication 10, **caractérisé en ce que** ledit transducteur ultrasonore (6) adopte un transducteur piézoélectrique avec une distance focale allant de 80 mm à 200 mm, un diamètre allant de 80 mm à 300 mm et une plage de fréquence de fonctionnement allant de 0.5 MHz à 2 MHz.
